# EUROPEAN PATENT SPECIFICATION

(11) **EP 0 579 323 B1**
(45) Date of publication and mention of the grant of the patent: **25.09.1996**
(21) Application number: 93202018.3
(22) Date of filing: 09.07.1993
(51) Int. Cl.: C07C 409/14, C07C 407/00

(54) **Process for the preparation of cyclohexyl hydroperoxide**
Verfahren zur Herstellung von Cyclohexylhydroperoxid
Procédé de préparation de cyclohexyl hydropéroxyde

(30) Priority: 15.07.1992 NL 9201269
(43) Date of publication of application: 19.01.1994
(73) Proprietor: DSM N.V., 6411 TE Heerlen (NL)
(72) Inventor: van de Moesdijk, Cornelis Gerardus Maria, NL-6176 BL Beek (L) (NL)

(56) References cited:
- BE-A- 660 522
- FR-A- 1 506 296

## Description

The invention relates to a process for the preparation of cyclohexyl hydroperoxide by converting cyclohexane into a mixture substantially consisting of 0.5-8 wt.% of cyclohexyl hydroperoxide and 0.1-4 wt.% of cyclohexanol and cyclohexanone in cyclohexane, this being effected with an oxygen-containing gas at a temperature between 130 and 200°C and a pressure between 4 and 50 bar during 0.05 to 14 hours in the absence of catalysts, and optionally subjecting the mixture after the reaction to partial expansion.

Such a process is known from EP-A-004105.

Cyclohexyl hydroperoxide is used for the preparation of cyclohexanol and cyclohexanone. To this end the cyclohexyl hydroperoxide is decomposed in a separate reaction step under the influence of a metal catalyst. The preparation of cyclohexanol and cyclohexanone through oxidation of cyclohexane to mainly cyclohexyl hydro-peroxide in the absence of catalysts and subsequent decomposition of the cyclohexyl hydroperoxide in a separate reaction step is called the uncatalyzed cyclo-hexane oxidation. It is essential that no (metal) catalysts be present during the oxidation.

In the catalyzed cyclohexane oxidation - where cobalt and/or chromium compounds are usually applied - cyclohexanol and cyclohexanone are the main products formed, besides a relatively small amount of cyclohexyl hydro-peroxide, a large portion of the cyclohexyl hydroperoxide being already decomposed during the oxidation. As a rule the product of the uncatalyzed oxidation comprises a weight percentage of cyclohexyl hydroperoxide that is at least comparable to the weight percentage of cyclohexanol + cyclohexanone. Often, the amount of cyclohexyl hydro-peroxide present in the mixture after reaction is 2x as large as the amount of cyclohexanol + cyclohexanone. In contrast, the catalyzed oxidation yields a mixture that contains less than 50% cyclohexyl hydroperoxide relative to the weight percentage of cyclohexanol + cyclohexanone. This is often even less than 40% peroxide compared with the weight percentage of cyclohexanol + cyclohexanone.

The advantage of the catalyzed oxidation is that this oxidation reaction can easily be carried out and simply be controlled. A disadvantage of the catalyzed oxidation is, however, that relatively many by-products are formed, such as alkyl carboxylic acids. In view of the low conversion in the cyclohexane oxidation (usually lower than 6%, but higher than 2%), this is a major disadvantage since a substantial part of the raw material is lost. Still, by far the majority of the commercial processes use a catalyzed oxidation.

In the uncatalyzed cyclohexane oxidation a smaller number of by-products is obtained. However, this process has the disadvantage that it is very difficult to start the oxidation reaction and to keep it going, so that a longer reaction time and/or higher temperatures are needed for an acceptable conversion, on account of which a part of the advantage is lost, for this also results in by-product formation.

The invention provides a solution to this problem and is characterized in that 0.1 to 3 wt.% of oxidic products with linear or cyclic alkyl chains with 1 to 6 carbon atoms are present in the cyclohexane at the start of the oxidation reaction.

GB-A-1151287 describes a process in which cyclohexane is oxidized in the presence of oxidic products. GB-A-1151287 however describes an oxidation of cyclohexane at 120°C and the oxidic products mentioned are products with the same carbon number as cyclohexane. Furthermore the continuous oxidation of cyclohexane takes place with 1.1 - 1.3 % cyclohexylhydroperoxide present and no indication is given how much cyclohexylhydroperoxide is present at the beginning of the oxidation. According to the present invention the start of the oxidation is promoted by adding extra oxidic components to the reaction mixture. In GB-A-1151287 the level of oxidic components is kept within certain range after the oxidation has started. The cyclohexylhydroperoxide in GB-A-1151287 has not been fed to the reaction mixture at the start of the oxidation but is cyclohexylhydroperoxide formed in situ.

In BE-A- 660.522 a process is described in which a cycloalkane with 8-16 carbon atoms is oxidized in the presence of an initiator such as cycloalkanoles, cycloalkanones and cycloalkylhydroperoxides. BE-A- 660.522 teaches however that the oxidation of cycloalkanes with high carbon numbers react different than cyclohexane.

It is surprising in view of an article of Vasin et al. (Khimicheskaya Promyshlennost, Vol. 20, No. 7, page 3-6, 1988) and in view of US-A-5043481 that adding C₆-oxidic products to the oxidation reaction of cyclohexane is advantageous. The article of Vasin et al. teaches that the concentration of cyclohexanol and cyclohexanone in the cyclohexane to be oxidized should be kept as low as possible because their presence would result in a loss of yield of useful products. US-A-5043481 teaches that the return of oxidation products to the oxidation zone results in significant loss of net selectivities on a molar basis to cyclohexylhydroperoxide, cyclohexanone and cyclo-hexanol. Thus US-A-5043481 teaches that the amount of C₆-oxidic products fed to the oxidation should be kept as low as possible. Furthermore US-A-5043481 and the literature cited there teach that it is desirable to add a tertiary alcohol in a large amount to the alkane to be oxidized in order to accelerate oxidation. This has as a disadvantage that makeup tertiary butanol, for example, is to be supplied continually.

From US-A-3109864 (which relates to a catalyzed oxidation process), for example, it appears that it is customary to purify the cyclohexane to be recycled.

The present invention provides a simplified process in which cyclohexane to be recycled need not be purified and no makeup of tertiary alcohols is needed, while still resulting in oxidized cyclohexane that is obtained at a high selectivity and a relatively high velocity.

Oxidic products with linear or cyclic alkyl chains are in particular alcohols, alkanals, alkene oxides, alkanones, acids and/or alkyl hydroperoxides. Preferably, use is made of alkanones, alkanols, alkanals and alkene oxides. Examples of suitable oxidic products are formic acid, acetic acid, ethanol, acetaldehyde, 1-propanol, 2-propanol, propanal, propanone, butanal, butanone, 1-butanol, 2-butanol, 2-pentanone, pentanal, pentanol, cyclopentanol, cyclohexanol, cyclohexanone, 6-hydroxy hexanone, 6-hydroxy hexanal, 1,2-dihydroxy cyclohexane, cyclohexyl hydroperoxide, cyclohexene oxide or mixtures of these.

As a rule the boiling points of the oxidic products are lower than that of cyclohexanone.

Preferably, use is made of mixtures of oxidic products, mixtures being simply available.

Linear or cyclic alcohols, alkanals, alkanones, alkene oxides, acids and/or alkyl hydroperoxides with 1-6 carbon atoms are preferably added to clean cyclohexane at the start-up of a plant. Use may also be made of cyclohexane in which these components are already present. In the rest of this application the word "components" will be used as a collective noun designating the oxidic products with linear or cyclic alkyl chains.

Preferably, more than 0.2 wt.% and in particular more than 0.25 wt.% of these oxidic products are present in the cyclohexane to be oxidized. As a rule the advantages of the invention are fully achieved if the amount of oxidic products is below 2 wt.%, the upper limit not being critical. However, if the amount of oxidic products is too high at the start, the process efficiency decreases because the maximum desirable conversion is rapidly achieved. The amount of C₆ components is preferably less than 1 wt.% and the amount of C₅ components is preferably less than 1 wt.%.

Oxidation can be performed either batch wise or continuously. In practice the oxidation is performed continuously in a serie of at least two reactor parts in which the amount of cyclohexyl hydroperoxide, cyclohexanol and cyclohexanone will increase in each reactor part until the required amount of these oxidic C₆ components is reached in the last reactor part. The start of the oxidation reaction according to the invention, takes place in the first reactor part or first reactor parts. The circumstances of oxidation in this reactor part can be compared to the start of a batch wise oxidation. The circumstances of oxidation in the last reactor can be compared to the circumstances at the end of a batch wise oxidation.

Oxidation preferably takes place in a system of reactors arranged in series or in a pipe-reactor with compartments. Usually oxygen or oxygen containing gases are supplied to each reactor or reactor part. It is very important that the oxidation starts in the first reactor(s) (reactor parts) because otherwise the off gases leaving the seperate reactors will contain too much oxygen and hydrocarbons (such as evaporated cyclohexane). These gas mixtures can cause explosions. By performing the oxidation according to the invention this safety risk can be minimized.

As a rule the reaction is carried out autothermally or through control of the temperature. Temperature control is usually effected by discharging the reaction heat via a gas flow, an intermediate cooling, or other methods known to one skilled in the art. To prevent transition metals (which promote cyclohexyl hydroperoxide decomposition) from entering the mixture to be oxidized, preferably reactors are chosen the internal walls of which are inert. Use can be made for example of reactors with internal walls made of passivated steel, aluminium, tantalum, glass or enamel. This is of particular importance for small production capacities, where the ratio between wall area and liquid volume is unfavourable. For large capacities, separate inertization of the wall is not strictly necessary. It will be clear that, if a negligible amount of metal ions not having any substantial influence on the reaction enters the oxidation mixture, in the framework of this invention the reaction is considered an uncatalyzed cyclohexane oxidation. In contrast with the uncatalyzed cyclohexane oxidation, the catalyzed cyclohexane oxidation - as a rule with cobalt and/or chromium addition - yields a reaction mixture with a relatively small amount of cyclohexyl hydroperoxide compared with cyclohexanol + cyclohexanone.

It is essential to the process according to the invention that the components be present already at the start of the oxidation reaction. In a continuously operated oxidation the components are fed to the (first) reactor part in which the start of the oxidation takes place. Although this is not necessary, preferably the components are added to the cyclohexane before the cyclohexane enters the (first) reactor, or the cyclohexane already contains these components. It is also possible for the components (optionally mixed with a portion of the required cyclo-hexane) to be passed through a separate line into the (first) reactor and to be mixed with clean cyclohexane there. Clean cyclohexane is understood to mean cyclohexane with less than about 0.05 wt.% of oxidic products with linear or cyclic alkyl chains.

The start of the oxidation according to the invention can be defined in two ways. First, the start of the oxidation takes place when between 0 and 0.5 wt.% oxidic C₆ components are formed apart from the oxidic C₆ components added according to the invention. Second, the start of the oxidation, regardless of the amount of oxidic components added according to the invention, takes place when oxygen and cyclo-hexane are present together at a certain temperature (130-200°C) during a certain residence time. At relatively low temperatures, like 160°C, the start of the oxidation will take place in the first 20 minutes. At higher temperatures, like 190°C, the start will take place in the first 2 minutes. By using the law of Arhenius one can now easily calculate during which period at a certain temperature the start of the oxidation takes place.

In a first embodiment of the process according to the invention use is made of 0.1 to 3 wt.% of components with 1-5 carbon atoms. These components with fewer than 6 carbon atoms are preferred because either they are discharged together with evaporating cyclohexane, or they can relatively easily be separated also upon further oxidation to acids, or they are relatively rapidly oxidized to CO₂ upon further oxidation, so that the mixture after reaction contains a relatively small amount of interfering component.

The use of these components is very advantageous, not only because they strongly increase the reaction velocity, but also because it was found that these components do not have any adverse influence on the selectivity of the oxidation process.

In a second preferred embodiment of the process according to the invention, besides more than 0.1 wt.% of components with 1 to 5 carbon atoms use is also made of more than 0.1 wt.% of components with 6 carbon atoms. This relates specifically to cyclohexanol, cyclohexanone and cyclohexyl hydroperoxide. Hexanal and cyclohexane epoxide are very suitable, too. Preferably, this C₆ component is present in an amount of less than 1 wt.%, since larger amounts decrease the selectivity towards the target product. This embodiment is advantageous because during cyclohexane oxidation the amount of components with 1-5 carbon atoms may become smaller than desirable. By keeping process-inherent C₆ component in the cyclohexane that is to be recycled, or optionally by making up for any losses, there is no need for the purchase or makeup of components with 1-5 carbon atoms.

In practice, particularly components with 4-5 carbon atoms - besides those with 6 - prove very suitable.

In a third embodiment of the process according to the invention use is made of 0.1-3 wt.% of C₆ component. Preferably use is made of less than 1 wt.% C₆ components. This is of special advantage if entirely 'clean' cyclohexane is used. The C₆ component can be obtained, for example, from the light components flow (consisting particularly of hexanal and cyclohexene oxide in cyclohexanone) that is distilled off in the cyclohexanone purification process. Until now burning of these components has been fairly customary.

For one skilled in the art it is also possible to combine these embodiments, so that independent of the process operation of the oxidation one or more measures can be applied simultaneously or independently.

In particular the second embodiment is preferably carried out by condensing the off-gases formed during oxidation, optionally together with the gas mixture formed upon expansion of the reaction mixture (together: the gas flow), and supplying them to the start of the uncatalyzed cyclohexane oxidation without any further purification step. This ensures that the desired amounts of C₁-C₆ components continue being recycled, and in the case of continuous process operation there is no need to add linear or cyclic oxidic products to the process to ensure that the oxidation reaction proceeds properly.

There are energetic advantages in using the heat released upon condensation of the recycled gas flow for heating the fresh cyclohexane fed to the reactor. This can be effected either using a heat exchanger or by direct absorption of the gas flow in cyclohexane that is to be heated.

As oxygen-containing gas, oxygen as such may be chosen, air, rich or poor in oxygen, or oxygen mixed with nitrogen or another inert gas. Air is preferred, but the air can be mixed with extra inert gas to eliminate the risk of explosions. To eliminate the risk of explosions usually so much oxygen-containing gas is fed to the reactors, in such a way, that the oxygen concentration of the off-gas remains below the explosive limit. The oxygen feed (calculated as pure oxygen) as a rule amounts to between 0.1 and 50 l (NTP) per l of cyclohexane. This amount depends on the reaction velocity, and preferably oxygen is present in a small excess, but this is not critical, the amount of oxygen as a rule not being a limiting factor.

Usually it is desirable to cool the reaction mixture leaving the oxidation reactor before allowing the cyclohexyl hydroperoxide to decompose. As a rule the reaction mixture is cooled at least 10°C, preferably at least 30°C. The reaction mixture is preferably cooled by expansion, but cooling can also be effected by means of heat exchangers. If expansion is applied, a portion of the cyclohexane is evaporated (with some C₅₋C₆ components), which is preferably fed back to the oxidation. On account of the expansion simultaneous concentration of the cyclohexyl hydroperoxide to be decomposed takes place. This is advantageous as it makes it possible to carry out the decomposition step more efficiently and because less decomposition catalyst (see below) is needed.

Cyclohexyl hydroperoxide decomposition takes place, after cooling, under the influence of a transition metal catalyst, such as cobalt or chromium, preferably as described in EP-A-004105 or EP-A-092867.

As a rule the decomposition temperature is between 20 and 150°C, preferably between 50 and 130°C. The pressure in this step is not critical and usually it will be between 1 and 30 bar.

Cyclohexyl hydroperoxide, preferably in a concentration of 4-50%, can also be applied very advantageously as oxidizing agent in the preparation of alkane oxide from a corresponding alkene.

The invention will be elucidated on the basis of the following non-restrictive examples.

### Examples

### Comparative Experiment A

A 5 l batch reactor with a reflux condenser was charged with pure cyclohexane (99.9+%, HPLC grade). The temperature was set at 165°C. The vapour from the reactor was completely condensed in the reflux condenser. Air was blown through the reactor and after 25 minutes the batch experiment was stopped. Subsequently, the liquid composition was analyzed. Besides cyclohexane there was only 0.26 mol% of cyclohexyl hydroperoxide, 0.02 mol% of cyclohexanol and 0.01 mol% of cyclohexanone. Acids and C₃₋ C₅ components were found only in trace amounts.

### Example I

A 5 l batch reactor with a reflux condenser was charged with cyclohexane. In total the cyclohexane contained 0.2 mol% of cyclohexyl hydroperoxide, cyclohexanol and cyclohexanone. By means of a thermostatted bath the reactor temperature was raised to 165°C and maintained at that level. Air was blown through the reactor. The vapour from the reactor was completely condensed in the reflux condenser. After 25 minutes the air supply was shut off and the reactor cooled. The liquid contained 1.89 mol% of cyclohexyl hydroperoxide, 0.22 mol% of cyclohexanone, 0.20 mol% of cyclohexanol, 0.18 mol% of acids and 0.08 mol% of components with 3-5 carbon atoms. This experiment proves that in this amount oxidic products with 6 carbon atoms have a strongly reaction accelerating effect.

### Example II

The reactor described in Example I was charged with cyclohexane containing 0.2 mol% of cyclohexanol, cyclohexanone and cyclohexyl hydroperoxide as well as 0.25 mol% of C₄ and C5 components (butanol, pentanone, pentanal and pentanol). The temperature was set at 165°C. After air had been blown through for 25 minutes, the reaction was stopped. The liquid was analyzed and it was found to contain 2.97 mol% of cyclohexyl hydroperoxide, 0.54 mol% of cyclohexanone, 0.43 mol% of cyclohexanol, 0.41 mol% of acid and 0.29 mol% of C₃₋C₅ components. This experiment proves that the addition of C₄/C₅ components strongly increases the reaction velocity.

### Example III

The experiment of Example II was repeated at 160°C. After 25 minutes the reaction was stopped. The liquid composition was: 2.03 mol% of cyclohexyl hydro-peroxide, 0.21 mol% of cyclohexanone, 0.19 mol% of cyclohexanol, 0.13 mol% of acids and 0.26 mol% of C₃-C₅ components. This experiment proves that at the same conversion as in Example I a much better selectivity was achieved (0.13 mol% of acid instead of 0.18 mol%) through the use of a reaction temperature that was 5°C lower.

### Example IV

The reactor was charged with cyclohexane containing 0.3 mol% of cyclohexanol, cyclohexanone and cyclohexyl hydroperoxide and 0.25 mol% of C₄₋C₅ components. The reactor temperature was raised to 160°C and air was blown through. After 25 minutes the reaction was stopped. The liquid was analyzed, yielding the following composition: 2.05 mol% of cyclohexyl hydroperoxide, 0.23 mol% of cyclohexanol, 0.27 mol% of cyclohexanone, 0.15 mol% of acids and 0.31 mol% of C₃₋C₅ components. This experiment proves that on account of addition of C₄₋C₅ components a larger amount of C6 component does not have any adverse influence on the overall selectivity as compared with Example II.

### Example V

The first reactor of a cascade of 4 reactors in series (25 l each) was charged with 141.6 kg/hr of cyclohexane. Besides cyclohexane this flow contained 0.12 wt.% of cyclohexyl hydroperoxide, 0.32 wt.% of cyclo-hexanol, 0.19 wt.% of cyclohexanone and 0.34 wt.% of C₁₋C₅ component. The reactors were continuously operated at a pressure of 9.7 bar and a temperature of 169-170°C. The overhead discharge flows from the reactors contained, besides nitrogen, 54 kg/hr of cyclohexane vapour with small amounts of C₁₋C₆ components was discharged. The oxidic products consisted mainly of butanol, 2-pentanone, pentanol, pentanal and cyclopentanol, i.e. mainly of C₄₋C₅ components, and hexanal and epoxy cyclohexane as C₆ component. This vapour was absorbed in 87.6 kg/hr of cold cyclohexane, and this mixture was used as feed for the first reactor. By recycling in this way the amount of oxidic products in the cyclohexane to be oxidized could be kept roughly constant, this amount being such that a uniform and smooth continuous reaction took place. To each of the reactors 1.6 kg/hr of air was supplied. The reaction, and in particular the residence time, was controlled such (by adjusting the cyclohexane feed flow) that slightly more than 2% cyclohexyl hydroperoxide was present in the reaction mixture discharged. Under these conditions the residence time was about 0.7 hours, and the flow leaving the 4th oxidation reactor 88.6 kg/hr. This flow contained 2.2 wt.% of cyclohexyl hydroperoxide, 1.6 wt.% of cyclohexanol, 0.76 wt.% of cyclohexanone, 0.4 wt.% of acid and 0.27 wt.% of C₃₋C₅ components. The efficiency in terms of the target product (cyclohexyl hydroperoxide, cyclohexanone and cyclohexanol) was 87 wt.%. Through expansion (evaporation, mainly of cyclohexane) the reaction mixture was cooled down to 110°C. The cyclohexyl hydroperoxide concentration in the mixture after cooling was 3.1 wt.%.

### Example VI

The first reactor of a set-up as described in Example V was charged with 181 kg/hr of cyclohexane. The composition of this flow was 0.12 wt.% of cyclohexyl hydroperoxide, 0.27 wt.% of cyclohexanol, 0.16 wt.% of cyclohexanone and 0.63 wt.% of C₁₋C₅ components. The oxidation reactors were operated as in Example V at 9.7 bar and 169-170°C. Besides nitrogen, the overhead flows from the reactors contained 66.5 kg/hr of cyclohexane vapour with small amounts of C₁₋C₆ components. This vapour was absorbed in 114.5 kg/hr of cold cyclohexane, and this mixture was used as feed for the first reactor. To each of the reactors 1.8 kg/hr of air was supplied. The reaction, and in particular the residence time, was controlled such (by adjusting the cyclohexane feed flow) that slightly more than 2% cyclohexyl hydroperoxide was present in the outflowing reaction mixture. Under these conditions the residence time was 0.55 hours, and the flow leaving the 4th reactor 115.5 kg/hr. This flow contained 2.3 wt.% of cyclohexyl hydroperoxide, 1.3 wt.% of cyclohexanol, 0.6 wt.% of cyclohexanone, 0.31 wt.% of acid and 0.28 wt.% of C₃₋C₅ compounds. The efficiency in terms of the target product (cyclohexyl hydroperoxide, cyclohexanone and cyclohexanol) was 88 wt.%.

This example proves that there is a clear increase in the reaction velocity upon an increase in the amount of C₃₋C₅ components from ∼ 0.3 to ∼ 0.6 wt.%, while the selectivity towards the target compounds is even enhanced somewhat.

In this example, too, it proved to be possible to carry out a smooth, uniform, continuous, uncatalyzed cyclohexane oxidation.

### Example VII

In the same way as in Example VI, 181 KG/HR of cyclohexane was supplied, which consisted of 90 kg/hr of clean cyclohexane, 66.5 kg/hr of vapour from the reactors, and 24.5 kg of vapour obtained upon expansion of the outflowing reaction mixture (which resulted in cooling of the mixture from 180°C to 110°C). The composition of the cyclohexane fed to the first reactor was: 0.19 wt.% of cyclohexyl hydroperoxide, 0.35 wt.% of cyclohexanol, 0.21 wt.% of cyclohexanone and 0.72 wt.% of C₃₋C₅ components. The flow leaving the fourth reactor amounted to 115 kg/hr. This flow contained 2.4 wt.% of cyclohexyl hydroperoxide, 1.5 wt.% of cyclohexanol, 0.8 wt.% of cyclohexanone, 0.42 wt.% of acid and 0.30 wt.% of C₃₋C₅ components.

This experiment shows that recycle flows from the process are very suitable for the supply of C₃₋C₅ components to cyclohexane.

### Example VIII

The cyclohexyl hydroperoxide containing flow obtained in Example VI was subjected to a peroxide decomposition step as described in Example I of EP-A-92867. The resulting mixture, mainly comprising cyclohexanol and cyclohexanone, was subjected to separation by distillation. In a first column the components lighter than cyclohexanone were separated off. These consisted of hexanal, 1,2-dihydroxy cyclohexane, epoxy cyclohexane and some cyclohexanone.

In the same way as in Example III cyclohexane was air-oxidized, 1.0 wt.% of the mixture from the above-described first column having been added to the clean cyclohexane (so that the cyclohexane contained 0.3 wt.% of C₅ and 0.6 wt.% of C₆ components). The composition of the liquid obtained after 25 minutes' oxidation was: 2.6 wt.% of cyclohexyl hydroperoxide, 0.47 wt.% of cyclohexanol, 0.74 wt.% of cyclohexanone, 0.41 wt.% of acids and 0.62 mol% of C₃₋C₅ components.

The examples prove that there are several points in a process for the preparation of cyclohexanone and cyclohexanol at which oxidic products are formed that can very advantageously be used in the process according to the invention.

## Claims

1. Process for the preparation of cyclohexyl hydro-peroxide by converting cyclohexane into a mixture consisting substantially of 0.5-8 wt.% of cyclohexyl hydroperoxide and 0.1-4 wt.% of cyclohexanol and cyclohexanone in cyclohexane, this being effected using an oxygen-containing gas at a temperature between 130 and 200°C and a pressure between 4 and 50 bar during 0.05 to 14 hours in the absence of catalysts, and optionally subjecting the mixture after the reaction to partial expansion, this process being characterized in that 0.1 to 3 wt.% of oxidic products with linear or cyclic alkyl chains with 1-6 carbon atoms are present in the cyclohexane at the start of the oxidation reaction.

2. Process according to claim 1, characterized in that the oxidation is performed continuously in a serie of at least two reactor parts in which the start of the oxidation reaction takes place in the first reactor part or first reactor parts and in which the amount of cyclohexyl hydroperoxide, cyclohexanol and cyclohexanone increases in every reactor part.

3. Process according to claim 2, characterized in that the oxidic products are fed to the reactor part in which the start of the oxidation takes place.

4. Process according to either of claims 1-3, characterized in that as oxidic products use is made of formic acid, acetic acid, ethanol, acetaldehyde, 1-propanol, 2-propanol, propanal, propanone, butanal, butanone, 1-butanol, 2-butanol, 2-pentanone, pentanal, pentanol, cyclo-pentanol, cyclohexanol, cyclohexanone, 6-hydroxy hexanone, 6-hydroxy hexanal, 1,2-dihydroxy cyclo-hexane, cyclohexyl hydroperoxide, cyclohexene oxide or mixtures of these.

5. Process according to either of claims 1-4, characterized in that more than 0.25 wt.% of oxidic products is present in the cyclohexane to be oxidized.

6. Process according to any one of claims 1-5, characterized in that the oxidic products originate from recyclable flows of a cyclohexane oxidation process.

7. Process according to any one of claims 1-6, characterized in that the mixture after the reaction contains more cyclohexyl hydroperoxide than cyclo-hexanol plus cyclohexanone (in wt.%).

8. Process for starting the uncatalyzed cyclohexane oxidation process according to any one of claims 1-7 by adding 0.1-3 wt.% of oxidic products with linear or cyclic alkyl chains with 1-6 carbon atoms to cyclohexane and subsequently passing an oxygen-containing gas through the cyclohexane mixture, the mixture being kept at a temperature between 130°C and 200°C and a pressure between 4 and 50 bar.

9. Process according to any one of claims 1-8, characterized in that 0.1-3 wt.% of linear or cyclic oxidic products with 1-5 carbon atoms is present in the cyclohexane to be oxidized.

10. Process according to any one of claims 1-8, characterized in that more than 0.1 wt.% of oxidic products with 1-5 carbon atoms and 0.1-1 wt.% of oxidic products with 6 carbon atoms are present in the cyclohexane to be oxidized.

11. Process according to any one of claims 1-8, characterized in that 0.1-3 wt.% of oxidic products with 6 carbon atoms is present in the cyclohexane to be oxidized.

12. Process according to claim 1 characterized in that 0.1 to 1 wt.% oxidic products with linear or cyclic alkyl chains with 6 carbon atoms are present in the cyclohexane at the start of the oxidation reaction.

13. Process according to claim 1 characterized in that 0.1 to 1 wt.% oxidic products with linear or cyclic alkyl chains with 1-5 carbon atoms are present in the cyclohexane at the start of the oxidation reaction.

14. Process according to either of claims 12-13, characterized in that the oxidation reaction is performed continuously in a serie of at least two reactor parts in which the start of the oxidation reaction takes place in the first reactor part or first reactor parts and in which the amount of cyclo-hexyl hydroperoxide, cyclohexanol and cyclohexanone increases in every reactor part.

## Patentansprüche

1. Verfahren zur Herstellung von Cyclohexylhydroperoxid durch das Überführen von Cyclohexan in eine Mischung, die im wesentlichen aus 0,5 bis 8 Masse-% Cyclohexylhydroperoxid und 0,1 bis 4 Masse-% Cyclohexanol und Cyclohexanon in Cyclohexan besteht, wobei dies unter Verwendung eines sauerstoffhaltigen Gases bei einer Temperatur zwischen 130 und 200°C und einem Druck zwischen 4 und 50 bar während 0,05 bis 14 Stunden in Abwesenheit von Katalysatoren bewirkt wird, und gegebenenfalls Unterwerfen der Mischung, nach der Reaktion, einer partiellen Expansion, welches Verfahren dadurch gekennzeichnet ist, daß 0,1 bis 3 Masse-% Oxid-Produkte mit linearen oder cyclischen Alkylketten mit 1 bis 6 Kohlenstoffatomen im Cyclohexan beim Start der Oxidationsreaktion vorliegen.

2. Verfahren nach Anspruch 1, dadurch gekennzeichnet, daß die Oxidation kontinuierlich in einer Serie von zumindest zwei Reaktorteilen durchgeführt wird, wobei der Start der Oxidationsreaktion im ersten Reaktorteil oder in den ersten Reaktorteilen stattfindet, und wobei die Menge an Cyclohexylhydroperoxid, Cyclohexanol und Cyclohexanon in jedem Reaktorteil zunimmt.

3. Verfahren nach Anspruch 2, dadurch gekennzeichnet, daß die Oxid-Produkte dem Reaktorteil zugeführt werden, in dem der Start der Oxidation stattfindet.

4. Verfahren nach einem der Ansprüche 1 bis 3, dadurch gekennzeichnet, daß als Oxid-Produkte Ameisensäure, Essigsäure, Ethanol, Acetaldehyd, 1-Propanol, 2-Propanol, Propanal, Propanon, Butanal, Butanon, 1-Butanol, 2-Butanol, 2-Pentanon, Pentanal, Pentanol, Cyclopentanol, Cyclohexanol, Cyclohexanon, 6-Hydroxyhexanon, 6-Hydroxyhexanal, 1,2-Dihydroxycyclohexan, Cyclohexylhydroperoxid, Cyclohexenoxid oder Mischungen hievon verwendet werden.

5. Verfahren nach einem der Ansprüche 1 bis 4, dadurch gekennzeichnet, daß mehr als 0,25 Masse-% Oxid-Produkte im zu oxidierenden Cyclohexan vorliegen.

6. Verfahren nach einem der Ansprüche 1 bis 5, dadurch gekennzeichnet, daß die Oxid-Produkte aus rückführbaren Strömen eines Cyclohexan-Oxidationsverfahrens stammen.

7. Verfahren nach einem der Ansprüche 1 bis 6, dadurch gekennzeichnet, daß die Mischung nach der Reaktion mehr Cyclohexylhydroperoxid als Cyclohexanol plus Cyclohexanon (in Masse-%) enthält.

8. Verfahren zum Starten des unkatalysierten Cyclohexan-Oxidationsverfahrens nach einem der Ansprüche 1 bis 7 durch den Zusatz von 0,1 bis 3 Masse-% Oxid-Produkten mit linearen oder cyclischen Alkylketten mit 1 bis 6 Kohlenstoffatomen zu Cyclohexan und anschließendes Führen eines sauerstoffhaltigen Gases durch die Cyclohexan-Mischung, wobei die Mischung bei einer Temperatur zwischen 130°C und 200°C und einem Druck zwischen 4 und 50 bar gehalten wird.

9. Verfahren nach einem der Ansprüche 1 bis 8, dadurch gekennzeichnet, daß 0,1 bis 3 Masse-% lineare oder cyclische Oxid-Produkte mit 1 bis 5 Kohlenstoffatomen im zu oxidierenden Cyclohexan vorliegen.

10. Verfahren nach einem der Ansprüche 1 bis 8, dadurch gekennzeichnet, daß mehr als 0,1 Masse-% Oxid-Produkte mit 1 bis 5 Kohlenstoffatomen und 0,1 bis 1 Masse-% Oxid-Produkte mit 6 Kohlenstoffatomen im zu oxidierenden Cyclohexan vorliegen.

11. Verfahren nach einem der Ansprüche 1 bis 8, dadurch gekennzeichnet, daß 0,1 bis 3 Masse-% Oxid-Produkte mit 6 Kohlenstoffatomen im zu oxidierenden Cyclohexan vorliegen.

12. Verfahren nach Anspruch 1, dadurch gekennzeichnet, daß 0,1 bis 1 Masse-% Oxid-Produkte mit linearen oder cyclischen Alkylketten mit 6 Kohlenstoffatomen im Cyclohexan beim Start der Oxidationsreaktion vorliegen.

13. Verfahren nach Anspruch 1, dadurch gekennzeichnet, daß 0,1 bis 1 Masse-% Oxid-Produkte mit linearen oder cyclischen Alkylketten mit 1 bis 5 Kohlenstoffatomen im Cyclohexan beim Start der Oxidationsreaktion vorliegen.

14. Verfahren nach Anspruch 12 oder 13, dadurch gekennzeichnet, daß die Oxidationsreaktion kontinuierlich in einer Serie von zumindest zwei Reaktorteilen durchgeführt wird, wobei der Start der Oxidationsreaktion im ersten Reaktorteil oder in den ersten Reaktorteilen stattfindet, und wobei die Menge an Cyclohexylhydroperoxid, Cyclohexanol und Cyclohexanon in jedem Reaktorteil zunimmt.

## Revendications

1. Procédé de préparation de cyclohexylhydroperoxyde en convertissant du cyclohexane en un mélange composé de façon substantielle de 0,5 à 8 % en masse de cyclohexylhydroperoxyde et de 0,1 à 4 % en masse de cyclohexanol et de cyclohexanone dans du cyclohexane, ceci étant effectué en utilisant un gaz contenant de l'oxygène à une température comprise entre 130 et 200° C et à une pression comprise entre 4 et 50 bars pendant 0,05 à 14 heures en l'absence de catalyseurs, et en option en soumettant le mélange après la réaction à dilatation partielle, ce procédé étant caractérisé en ce que 0,1 à 3 % en masse de produits d'oxydation présentant des chaînes alkyle cycliques ou linéaires ayant 1 à 6 atomes de carbone sont présents dans le cyclohexane au démarrage de la réaction d'oxydation.

2. Procédé selon la Revendication 1, caractérisé en ce que l'oxydation est effectuée en continu dans une série d'au moins deux parties de réacteur dans lesquelles le démarrage de la réaction d'oxydation a lieu dans la première partie de du réacteur ou les premières parties du réacteur, dans lesquelles la quantité de cyclohexylhydroperoxyde, de cyclohexanol et de cyclohexanone augmente dans chaque partie du réacteur.

3. Procédé selon la Revendication 2, caractérisé en ce que les produits d'oxydation sont chargés dans la partie de réacteur dans laquelle le démarrage de l'oxydation a lieu.

4. Procédé selon l'une quelconque des Revendications 1 à 3, caractérisé en ce que, comme produits d'oxydation, on utilise de l'acide formique, de l'acide acétique, de l'éthanol, de l'acétaldéhyde, du 1-propanol, du 2-propanol, du propanal, du propanone, du butanal, du butanone, du 1-butanol, du 2-butanol, du 2-pentanone, du pentanal, du pentanol, du cyclo-pentanol, du cyclohexanol, du cyclohexanone, du 6-hydroxy-hexanone, du 6-hydroxy-hexanal, du 1,2-dihydroxycyclohexane, du cyclohexylhydroperoxyde, de l'oxyde de cyclohexène ou des mélanges de ceux-ci.

5. Procédé selon l'une des Revendications 1 à 4, caractérisé en ce que plus de 0,25 % en masse de produits d'oxydation sont présents dans le cyclohexane à oxyder.

6. Procédé selon l'une quelconque des Revendications 1 à 5, caractérisé en ce que les produits d'oxydation ont pour origine des flux recyclables d'un procédé d'oxydation du cyclohexane.

7. Procédé selon l'une quelconque des Revendications 1 à 6, caractérisé en ce que le mélange après la réaction contient plus de cyclohexylhydroperoxyde que de cyclohexanol plus cyclohexanone (pourcentage en masse).

8. Procédé destiné à démarrer le procédé d'oxydation de cyclohexane non catalysé selon l'une quelconque des Revendications 1 à 7 en ajoutant 0,1 à 3 % en masse de produits d'oxydation présentant des chaînes alkyle cycliques ou linéaires présentant 1 à 6 atomes de carbone au cyclohexane et en passant ultérieurement un gaz contenant de l'oxygène dans le mélange de cyclohexane, le mélange étant maintenu à une température comprise entre 130° C et 200° C et à une pression comprise entre 4 et 50 bars.

9. Procédé selon l'une quelconque des Revendications 1 à 8, caractérisé en ce que 0,1 à 3 % en masse de produits d'oxydation cycliques ou linéaires présentant 1 à 5 atomes de carbone sont présents dans le cyclohexane à oxyder.

10. Procédé selon l'une quelconque des Revendications 1 à 8, caractérisé en ce que plus de 0,1 % en masse de produits d'oxydation présentant 1 à 5 atomes de carbone et 0,1 à 1 % en masse de produits d'oxydation présentant 6 atomes de carbone sont présents dans le cyclohexane à oxyder.

11. Procédé selon l'une quelconque des Revendications 1 à 8, caractérisé en ce que 0,1 à 3 % en masse des produits d'oxydation présentant 6 atomes de carbone sont présents dans le cyclohexane à oxyder.

12. Procédé selon la Revendication 1, caractérisé en ce que 0,1 à 1 % en masse de produits d'oxydation présentant des chaînes alkyle cycliques ou linéaires présentant 6 atomes de carbone sont présents dans le cyclohexane au démarrage de la réaction d'oxydation.

13. Procédé selon la Revendication 1, caractérisé en ce que 0,1 à 1 % en masse de produits d'oxydation présentant des chaînes alkyle cycliques ou linéaires présentant 1 à 5 atomes de carbone sont présents dans le cyclohexane au démarrage de la réaction d'oxydation.

14. Procédé selon l'une des Revendications 12 à 13, caractérisé en ce que la réaction d'oxydation est effectuée en continu dans une série d'au moins deux parties de réacteur, dans lequel le démarrage de la réaction d'oxydation a lieu dans la première partie du réacteur ou les premières parties de réacteur, et dans lequel la quantité de cyclohexylhydroperoxyde, de cyclohexanol et de cyclohexanone augmente dans chaque partie de réacteur.
